# EUROPÄISCHE PATENTSCHRIFT

(11) **EP 1 764 010 B1**
(45) Veröffentlichungstag und Bekanntmachung des Hinweises auf die Patenterteilung: **24.08.2016**
(21) Anmeldenummer: 06012203.3
(22) Anmeldetag: 14.06.2006
(51) Int. Cl.: A45D 26/00, A61B 17/54, B26B 19/48

(54) **Hautbearbeitungswerkzeug zum Hautpeeling, Verfahren zur Herstellung eines solchen Hautbearbeitungswerkzeugs und Verwendung eines solchen Hautbearbeitungswerkzeugs an einem Elektrohandgerät des persönlichen Bedarfs**
Skin peeling device,method for manufacturing such a device and use of such a device in a hand held electrical appliance for personal care
Dispositif de dermabrasion, procédé de fabrication d'un tel dispositif et utilisation d'un tel dispositif dans un appareil manuel électrique de soin personnel

(30) Priorität: 19.09.2005 DE 102005044737
(43) Veröffentlichungstag der Anmeldung: 21.03.2007
(73) Patentinhaber: Braun GmbH, 61476 Kronberg (DE)
(72) Erfinder: Amsel, Klaus, 61389 Schmitten (DE); Grieshaber, Frieder, 61267 Neu Anspach (DE); Lopez, Javier Perez, 65760 Eschborn (DE)

(56) Entgegenhaltungen:
- WO-A-00/45728
- WO-A-94/04116
- WO-A-03/051154
- WO-A-2006/031413
- WO-A2-03/051284
- DE-A1- 19 954 159
- JP-A- H0 578 694
- US-A- 5 960 506
- US-B1- 6 299 620

## Beschreibung

Die vorliegende Erfindung betrifft ein Hautbearbeitungswerkzeug zum Hautpeeling mit zumindest einem Bearbeitungskorpus, der eine Bearbeitungsfläche, insbesondere Schleiffläche, mit einer Vielzahl von Bearbeitungsvorsprüngen aufweist. Die Erfindung betrifft weiterhin ein Verfahren zur Herstellung eines solchen Hautbearbeitungswerkzeugs sowie ein Elektrohandgerät des persönlichen Bedarfs, ausgebildet als Haarentfernungs- und/oder - schnittgerät mit einem solchen Hautbearbeitungswerkzeug.

Beim sogenannten Hautpeeling werden regelmäßig Schleifwerkzeuge, wie beispielsweise Bimssteine oder schleifpapierähnliche Schleifkörper, verwendet, um abgestorbene oder lose Hautpartikel und -schüppchen abzutragen, Haarstoppel abzureiben und zu runden, aber auch die Haut zu stimulieren, um eine bessere Durchblutung zu erreichen. Bisweilen kann es hierbei jedoch auch zu Hautreizungen kommen, insbesondere wenn das verwendete Schleifwerkzeug zu rauh ist.

In der US 2,936,768 ist ein elektrisches Manikürgerät beschrieben, das einen Doppelarbeitskopf mit zwei jeweils oszillierend antreibbaren Arbeitseinheiten aufweist. Dabei wird in einer Ausführungsform vorgeschlagen, zwei unterschiedlich rauhe bzw. unterschiedlich stark abrasive Bearbeitungskörper an dem Bearbeitungskopf des Geräts vorzusehen. Konkret soll der eine Bearbeitungskörper für den Grobabtrag als Stahlfeile ausgebildet sein, während der Bearbeitungskörper für den Feinabtrag ein feineres Sandpapier sein soll. Diese vorbekannte Gerätschaft erlaubt zwar ein unterschiedlich starkes Schleifen bzw. Polieren der Fingernägel, die vorgenannten Probleme beim Hautpeeling kann sie jedoch nicht lösen.

In der US 6,299,620 B1 wird ein Gerät und ein Verfahren zur Entfernung von Epidermisschichten beschrieben. Das Gerät hat eine Haut-Kontaktfläche, bei der Diamantpartikel in einem Silikonmaterial eingelagert sind. Um entfernte Hautpartikel aus den Zwischenräumen zwischen den Diamantpartikeln fern zu halten, wird über Zuführungskanäle bei der Verwendung des Gerätes eine sterile Flüssigkeit zur Haut-Kontaktfläche geleitet, die die Hautpartikel abspült.

Der vorliegenden Erfindung liegt daher die Aufgabe zugrunde, ein verbessertes Hautbearbeitungswerkzeug der genannten Art, ein hierdurch verbessertes Elektrohandgerät der genannten Art sowie ein Verfahren zur Herstellung eines solchen Hautbearbeitungswerkzeugs zu schaffen, die Nachteile des Standes der Technik vermeiden und letzteren in vorteilhafter Weise weiterbilden. Insbesondere soll das Hautbearbeitungswerkzeug ein schonenderes, die Haut besser pflegendes Hautpeeling ermöglichen.

Erfindungsgemäß wird die genannte Aufgabe durch ein Hautbearbeitungswerkzeug gemäß Anspruch 1, ein Elektrohandgerät gemäß Anspruch 14 sowie ein Verfahren zur Herstellung eines Hautbearbeitungswerkzeugs gemäß Anspruch 16 gelöst. Bevorzugte Ausgestaltungen der Erfindung sind Gegenstand der abhängigen Ansprüche.

Es wird also vorgeschlagen, die Peelingbehandlung der Haut zum Aufbringen eines Applikationsstoffs auf die Haut zu nutzen. Durch den Hautschüppchenabtrag und die Hautstimulation beim Peeling ist die behandelte Haut besonders aufnahmebereit für Applikationsstoffe, die hierdurch eine besondere Wirksamkeit entfalten können und auf die gerade freigelegte, frische Hautschicht einwirken können. Erfindungsgemäß besitzt das Hautbearbeitungswerkzeug eine Applikationseinrichtung zum Aufbringen eines Applikationsstoffs auf die von dem Bearbeitungskorpus bearbeitbare Haut. Durch den Applikationsstoffauftrag beim Peeling kann eine schonendere Hautbehandlung erreicht werden. Der aufgebrachte Applikationsstoff vermindert durch den Hautpartikelabtrag möglicherweise verursachte Hautreizungen und pflegt die Haut zusätzlich, wobei durch den Applikationsstoffauftrag beim Peeling ein besonders effizientes Einziehen erreichbar ist.

Die Applikationseinrichtung kann grundsätzlich verschieden ausgebildet und angeordnet sein, wobei sie vorteilhafterweise in Weiterbildung der Erfindung derart ausgebildet ist, dass die Abgabe des Applikationsstoffs im Bereich der Bearbeitungsfläche erfolgt. Vorzugsweise gibt die Applikationseinrichtung den Applikationsstoff unmittelbar auf der Bearbeitungsfläche ab, mit der das Hautpeeling bewerkstelligt wird.

Die Applikationseinrichtung weist hierzu einen Applikationsstoffspeicher in und/oder auf der Benutzungsfläche des Bearbeitungskorpus auf. Die Abgabe des Applikationsstoffes kann unmittelbar aus dem Applikationsstoffspeicher durch Abrieb erfolgen, wenn der Applikationsstoffspeicher mit der bearbeiteten Haut in Berührung kommt, und/oder auch durch ein Ausbrechen einzelner Bearbeitungsvorsprünge herbeigeführt werden, die sich durch Verschleiß der Bearbeitungsfläche ergeben. Durch die Anordnung des Applikationsstoffspeichers in bzw. auf der Benutzungsoberfläche kann die Abgabe des Applikationsstoffes unmittelbar bei der Hautbearbeitung erfolgen.

In Weiterbildung der Erfindung ist der Applikationsstoff insbesondere in Oberflächensenken, insbesondere in Poren, zwischen den Bearbeitungsvorsprüngen der Bearbeitungsfläche eingelagert. Die poröse Bearbeitungsfläche ist sozusagen in ihren Poren mit dem Applikations-stoff beschichtet. Dies bewirkt nicht nur einen kontinuierlichen Applikationsstoffaustrag über längere Zeit, sondern besitzt auch den Vorteil, dass die Poren bzw. Vertiefungen in der Bearbeitungsoberfläche verschlossen werden, so dass sich dort keine Bakterien oder Verschmutzungen einnisten können. Grundsätzlich könnte die Applikationsstoffbeschichtung auch die Bearbeitungsvorsprünge erfassen bzw. überdecken. Um die Wirksamkeit der Bearbeitungsvorsprünge für den Peeling-Vorgang jedoch nicht zu beeinträchtigen, sind nach einer bevorzugten Ausführung der Erfindung die Applikationsstoffeinlagerungen im wesentlichen auf die Poren in der Bearbeitungsfläche beschränkt.

Grundsätzlich können verschiedene Applikationsstoffe Verwendung finden. In Weiterbildung der Erfindung kann der Applikationsstoff ein Hautpflegemittel beinhalten, das Feuchtigkeitsspender, Öle, Aloe Vera oder Coenzyme wie das Coenzym Q10 enthalten kann. Alternativ oder zusätzlich kann der Applikationsstoff auch einen antibakteriellen Wirkstoff enthalten, um die nach dem Hautpartikelabtrag empfindliche Haut vor Bakterienangriffen zu schützen.

Grundsätzlich wäre es denkbar, den Applikationsstoff auch aktiv auszubringen, beispielsweise Fördermittel vorzusehen, die den Applikationsstoff aktiv auf die Haut ausbringen. Vorzugsweise jedoch erfolgt ein passiver Applikationsstoffauftrag auf die Haut dadurch, dass der Applikationsstoff abreibbar auf die Bearbeitungsfläche aufgebracht ist, so dass der bei der Peelingbearbeitung entstehende Abrieb den eingelagerten Applikationsstoff nach und nach auf die Haut bringt.

Die Bearbeitungsfläche des Bearbeitungswerkzeugs kann in verschiedener Weise abrasiv wirkend ausgebildet sein. Beispielsweise könnte die Bearbeitungsfläche nach Art einer Feile und/oder einer Raspel ein regelmäßiges Raster von regelmäßig ausgebildeten Eingriffsvorsprüngen aufweisen, wobei auch hier in den Senken zwischen den Eingriffsvorsprüngen der Applikationsstoff eingelagert sein kann. Vorzugsweise jedoch ist die Bearbeitungsfläche als Schleifoberfläche ausgebildet, die eine Vielzahl von unregelmäßig geformten Schleifvorsprüngen besitzt, die nicht auf den Rasterpunkten eines streng geometrischen Rasters, sondern nach dem Zufallsprinzip angeordnet sind, wobei sich nichtsdestotrotz insgesamt natürlich trotzdem eine gleichmäßige Verteilung der Schleifvorsprünge ergibt.

In Weiterbildung der Erfindung kann die Schleifoberfläche dabei schleifpapierähnlich ausgebildet sein. Auf einem Träger des Bearbeitungskorpus können eine Vielzahl von Schleifkörnern befestigt, insbesondere festgeklebt sein. Die Schleifkörnerschicht kann dabei unmittelbar auf den Träger des Bearbeitungskorpus aufgebracht sein. Alternativ zu einer solchen direkten, unmittelbar am Träger befestigten Schleifkornschicht, bei der Zwischenträgerschichten entfallen können, kann jedoch auch vorgesehen sein, ein Schleifpapier auf dem Träger zu befestigen, d.h. die Schleifkörner auf einer Papierlage zu befestigen, insbesondere festzukleben und die Papierschicht dann auf dem Träger zu befestigen. Vorteilhafterweise kann der Träger für die Schleifkornschicht bzw. Schleifpapierschicht aus Kunststoff bestehen.

Alternativ oder zusätzlich zu einer solchen schleifpapierähnlichen Bearbeitungsfläche kann der Bearbeitungskorpus auch eine Schleifoberfläche besitzen, die als Plasmabeschichtung mit einem Keramik- und/oder Metallpulver ausgebildet ist. Alternativ oder zusätzlich kann auch eine Schleifoberfläche vorgesehen sein, die als Flammbeschichtung ausgebildet ist, wobei vorteilhafterweise ebenfalls ein Keramik- und/oder ein Metallpulver verwendet werden kann.

Die Rauheit der Bearbeitungsfläche kann je nach Anwendungsfall und für verschiedene Hauttypen variieren. Vorteilhafterweise bewegt sich die Rauhigkeit der Bearbeitungsoberfläche dabei in einem Bereich von 5 Rz bis 100 Rz, wobei sich für ein effizientes Hautpeeling bei gleichzeitigem Applikationsstoffauftrag eine Rauhtiefe von 30 Rz bis 70 Rz besonders eignet.

Ist die Bearbeitungsfläche in der vorgenannten Weise schleifpapierähnlich ausgebildet, kann das verwendete Schleifkorn in Weiterbildung der Erfindung eine Körnung zwischen 100 bis 1200 aufweisen. Die Körnung ist dabei ein Maß für die Feinheit eines Schleifpapiers, wobei die Körnungszahl die Maschenzahl je 25,4 mm Länge einer Siebseite eines Rüttelsiebs angibt. Das Schleifkorn der Körnung 100 besteht demgemäß aus Körnern mit einer Maximalkorngröße, die noch durch ein solches Rüttelsieb mit 100 Maschen je 25,4 mm Siebseitenlänge hindurchfallen.

In bevorzugter Ausgestaltung der Erfindung bildet das Hautbearbeitungswerkzeug ein Elektrohandgerät bzw. einen Teil hiervon. Das Hautbearbeitungswerkzeug wird als Geräteaufsatz für ein elektrisches Handgerät des persönlichen Bedarfs ausgebildet, welches wahlweise mit dem aufgesetzten Geräteaufsatz oder ohne den Geräteaufsatz betreibbar ist. Das Hautbearbeitungswerkzeug bildet eine Geräteaufsatz für ein Haarentfernungs- und/oder -schnittgerät, wie beispielsweise ein Epiliergerät oder ein Rasiergerät.

Der Geräteaufsatz besitzt dabei geeignete Befestigungsmittel zu seiner Befestigung an dem jeweiligen Gerät, wobei vorzugsweise aufsatzseitige Befestigungsmittel mit geräteseitigen Befestigungsmitteln, beispielsweise in Form von Rastverschlüssen, zusammenwirken. Der Geräteaufsatz besitzt formschlüssig wirkende Befestigungsmittel, die an geräteseitige Befestigungsmittel angepasst sind. Insbesondere kann der Geräteaufsatz in seiner Form insgesamt an den Bearbeitungskopf des Handgeräts angepasst sein, so dass er auf diesen aufgesteckt und dort verrastet werden kann.

Die Peelingwirkung der zumindest einen Bearbeitungsfläche des Werkzeugs sowie der dieser zugeordneten Applikationseinrichtung kann grundsätzlich durch händisches Hinwegbewegen der Bearbeitungsfläche über den jeweiligen Hautabschnitt erreicht werden. In Weiterbildung der Erfindung jedoch ist der die zumindest eine Bearbeitungsfläche aufweisende Bearbeitungskorpus des Werkzeugs fremdenergiebetätigt antreibbar. Der Bearbeitungskorpus ist hierzu in geeigneter Weise beweglich gelagert und mit einem vorzugsweise elektrischen Antrieb verbindbar, der die entsprechende Antriebsbewegung des Bearbeitungskorpus bewirkt. Ist das Werkzeug in der genannten Weise als Geräteaufsatz ausgebildet, ist der Bearbeitungskorpus vorteilhafterweise an einem Aufsatzgehäuse gelagert, wobei der Bearbeitungskorpus Kupplungsmittel zum Ankuppeln an einen Geräteantrieb des Handgeräts aufweist, so dass kein eigener Antrieb für den Bearbeitungskorpus vorgesehen werden muss.

Die Antriebsbewegung des Bearbeitungskorpus mit der zumindest einen Bearbeitungsfläche kann in kinematischer Hinsicht verschieden ausgebildet sein, allerdings wird vorzugsweise eine oszillierende Antriebsbewegung eingesetzt, um Reaktionskräfte und -momente zu vermeiden, die in Führen des Geräts erschweren würden. Insbesondere kann bei einer insgesamt länglichen Ausbildung der Bearbeitungsfläche eine translatorisch oszillierende Antriebsbewegung im wesentlichen parallel zur Längsachse der Bearbeitungsfläche vorgesehen werden. Alternativ kann jedoch auch eine rotatorisch oszillierende Antriebsbewegung vorgesehen sein, die den Bearbeitungskorpus über einen vorzugsweise recht kleinen Winkelbereich vor und zurück verdreht.

Der vorgenannte Applikationsstoff kann grundsätzlich in verschiedener Weise auf die bzw. in die Bearbeitungsfläche gebracht werden. Nach einer vorteilhaften Ausführung der Erfindung kann der Applikationsstoff durch Sprühtrocknen einer den Applikationsstoff enthaltenden Emulsion oder Dispersion auf die bzw. in die Bearbeitungsfläche gebracht werden. Wird der Applikationsstoff in einer Emulsion oder in flüssigem Zustand in die bzw. auf die poröse Bearbeitungsoberfläche gebracht, kann er von dem vorzugsweise hydrophilen Körper aufgenommen werden. Alternativ zu einem Aufsprühen des Applikationsstoffes kann der Applikationsstoffauftrag auch durch Tauchen erfolgen. Insbesondere bei einem schleifpapierähnlichen Aufbau der Schleifschicht des Bearbeitungskorpus kann der Applikationsstoffauftrag auch mittels einer Auftragswalze oder durch Printing erfolgen.

Weitere Merkmale, Vorteile, Ziele und Anwendungsmöglichkeiten der vorliegenden Erfindung oder spezieller Ausführungsformen hiervon ergeben sich aus der nachfolgenden Beschreibung der Ausführungsbeispiele. Dabei bilden alle beschriebenen oder bildlich dargestellten Merkmale für sich oder in beliebiger Kombination und Unterkombination den Gegenstand der vorliegenden Erfindung, auch unabhängig von ihrer Zusammenfassung in den Ansprüchen oder deren Rückbeziehung. In den Zeichnungen zeigen:
- Fig. 1:: ein Elektrohandgerät in Form eines Haarentfernungsgeräts mit einem als Geräteaufsatz ausgebildeten Hautbearbeitungswerkzeug nach einer bevorzugten Ausführung der Erfindung, wobei der Geräteaufsatz und das Handgerät in voneinander getrenntem Zustand gezeigt sind,
- Fig. 2:: das Handgerät aus Fig. 1 mit dem darauf aufgesetzten Hautbearbeitungswerkzeug-Aufsatz aus Fig. 1,
- Fig. 3:: eine vergrößerte perspektivische Ansicht des Geräteaufsatzes aus den vorhergehenden Figuren,
- Fig. 4:: einen Schnitt durch den Bearbeitungskopf des Handgeräts und des darauf aufgesetzten Aufsatzes aus den vorhergehenden Figuren,
- Fig. 5:: eine schematische Darstellung eines Bearbeitungskorpus mit einer Plasmabeschichtung, und
- Fig. 6:: eine schematische Darstellung eines Bearbeitungskorpus mit einer auf einen Träger aufgebrachten Schleifkörnerschicht.

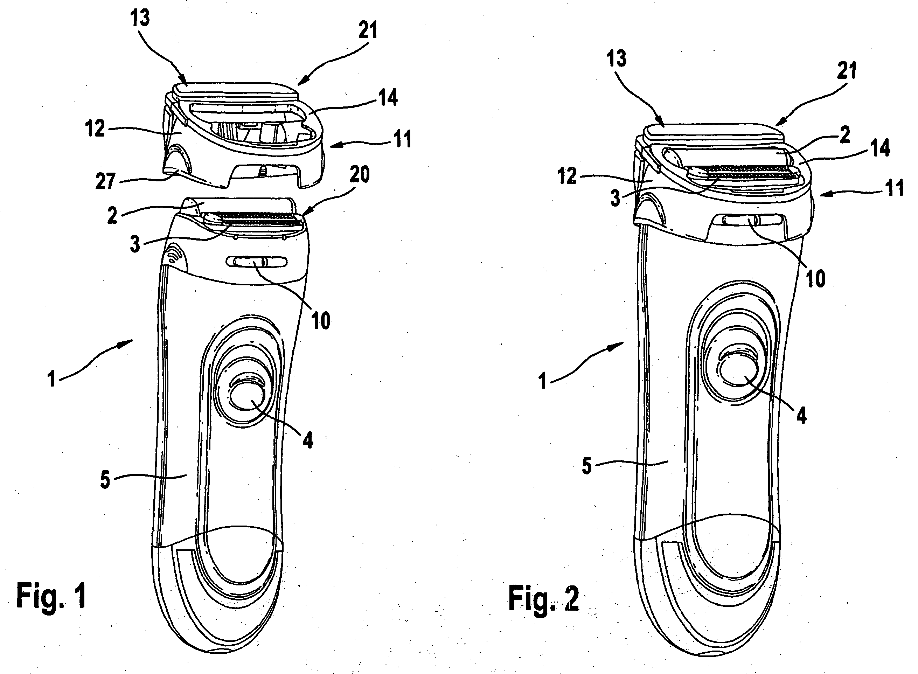

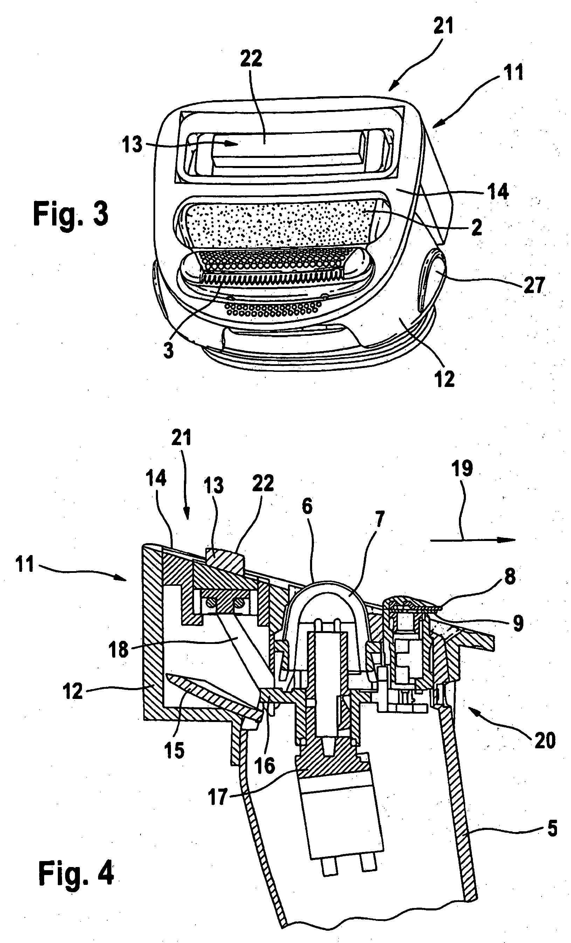

Die Figuren 1 und 2 zeigen ein elektrisches Haarentfernungsgerät 1 mit einem Kurzhaarschneider 2 und einem Langhaarschneider 3, die ebenso wie ein Betätigungsschalter 4 an einem Gehäuse 5 angeordnet sind. In dem genannten Gehäuse 5 ist in an sich bekannter Weise eine Stromversorgung und ein Elektromotor vorgesehen, die dem Antrieb des Kurzhaarschneiders 2 und des Langhaarschneiders 3 dienen, was jedoch aus Gründen der Übersichtlichkeit zeichnerisch nicht dargestellt ist. Der Kurzhaarschneider 2 kann in an sich bekannter Weise aus einer das Obermesser bildenden Scherfolie 6 und einem Untermesser 7 bestehen, das eine Vielzahl von Klingen besitzt und oszillierend unter der Scherfolie 6 bewegbar ist (vgl. Figur 4). Der Langhaarschneider 3 wiederum besitzt einen stationären Scherkamm 8, der das Obermesser bildet, und ein Untermesser, welches durch eine oszillierend antreibbare Scherklinge 9 gebildet ist, vgl. ebenfalls Figur 4.

Der Langhaarschneider 3 ist insgesamt elastisch nach oben vorgespannt und schwimmend gelagert, so dass er leicht der Hautkontur folgen kann. Er kann jedoch durch den Verriegelungsschalter 10 festgesetzt werden, so dass er besser für die Trimmfunktion eingesetzt werden kann.

Auf dem den Kurzhaarschneider 2 und den Langhaarschneider 3 aufweisenden Bearbeitungskopf 20 des Haarentfernungsgeräts 1 kann das als Geräteaufsatz 11 ausgebildete Hautbearbeitungswerkzeug 21 aufgesetzt werden. Wie Figur 3 zeigt, umfasst der Geräteaufsatz 11 einen im wesentlichen napfförmigen bzw. ringförmigen Aufsatzkorpus 12, an dem stirnseitig zur Außenseite freiliegend ein Bearbeitungskorpus 13 beweglich gelagert ist. In der gezeichneten Ausführung ist der Bearbeitungskorpus 13 insgesamt länglich und etwa plattenförmig ausgebildet und an dem Aufsatzkorpus 12 derart beweglich gelagert, dass er entlang seiner Längsachse, die etwa parallel zu dem Kurzhaarschneider 2 verläuft, oszillierend antreibbar ist.

Wie die Figuren 1 und 3 zeigen, kann der Aufsatzkorpus 12 über den Bearbeitungskopf 20 des Haarentfernungsgeräts 1 gestülpt werden, so dass er mit dem Gehäuse 5 des Haarentfernungsgeräts 1 fest verbunden ist. Der Aufsatzkorpus 12 besitzt dabei an seiner Stirnseite geeignete Aussparungen, so dass der Kurzhaarschneider 2 und der Langhaarschneider 3 auch bei aufgesetztem Geräteaufsatz 11 frei zugänglich liegen. Sowohl der Kurzhaarschneider 2 als auch der Langhaarschneider 3 überragen bei montiertem Geräteaufsatz 11 die an dem als Tragkörper fungierenden Aufsatzkorpus 12 ausgebildete, im wesentlichen ringförmig umlaufende Anlagefläche 14 nach oben, vgl. Figur 2 und 4. Die Innenkontur des Aufnahmekorpus 12 ist dabei an die Außenkontur des Bearbeitungskopfes 20 bzw. des Gehäuses 5 angepasst, so dass der Aufsatzkorpus 12 formschlüssig und kraftschlüssig auf dem Haarentfernungsgerät 1 sitzt. Vorteilhafterweise umfasst der Aufsatzkorpus 12 dabei elastische Verriegelungsmittel 27, insbesondere Rastmittel, die mit komplementären Verriegelungsmitteln, insbesondere Rastmitteln am Bearbeitungskopf 20 und/oder dem Gehäuse 5, verriegelbar sind.

Wie Figur 4 zu entnehmen ist, kann der Bearbeitungskorpus 13 durch eine im wesentlichen gabelförmige Kupplung 16 an den Antrieb des Haarentfernungsgeräts 1 angekuppelt werden. Im Bereich des Bearbeitungskopfes 20 ist an der Rückseite des Haarentfernungsgeräts 1 eine Klappe 15 angeordnet, die um eine Achse parallel zur Längserstreckung des Kurzhaarschneiders 2 bzw. des Bearbeitungskorpus 13 verschwenkt werden kann und hierdurch ein Abdecken bzw. Freilegen der genannten gabelförmigen Kupplung 16 ermöglicht, die selbst innerhalb des Gehäuses 5 vorgesehen und mit einer Schwingbrücke 17 des Antriebs der Scherelemente 7 bzw. 9 verbunden ist, vgl. Figur 4. Die Klappe 15 ist vor dem Aufsetzen des Geräteaufsatzes 11 zu öffnen, damit beim Aufsetzen der mit dem Bearbeitungskorpus 13 verbundene Kupplungsfinger 18 in Eingriff mit der gabelförmigen Kupplung 16 gebracht werden kann. Sobald dieser Eingriff hergestellt ist, kann die oszillierende Antriebsbewegung der Schwingbrücke 17 über die Kupplung 16 und den Kupplungsfinger 18 auf den Bearbeitungskorpus 13 des Hautbearbeitungswerkzeugs 21 übertragen werden, der hierzu entlang seiner Längsachse verschiebbar im Aufsatzkorpus 12 gelagert ist. Vorteilhafterweise ist durch eine entsprechende Ausgestaltung der Klappe und Abstimmung der geometrischen Verhältnisse mit dem Kupplungsfinger 18 bzw. dem Aufsatzkorpus 12 dafür gesorgt, dass sich die Klappe 15 beim Aufsetzen des Geräteaufsatzes 11 auf das Haarentfernungsgerät 1 automatisch öffnet.

Wird nun das Haarentfernungsgerät 1 mit aufgesetztem Hautbearbeitungswerkzeug 21 mit der zu bearbeitenden Hautfläche in Kontakt gebracht und in Vorschubrichtung 19 (vgl. Figur 4) über die Haut geführt, so werden zunächst durch den Langhaarschneider 3 auf der Haut befindliche längere Haare gekürzt, die dann vom nachfolgenden Kurzhaarschneider 2 ausrasiert werden. Schließlich wird die rasierte Haut durch den sich oszillierend hin- und herbewegenden Bearbeitungskorpus 13 endgültig geglättet, wobei nach Art einer Peelingbehandlung auch ausgetrocknete oder abgestorbene Hautpartikel oder Verhornungen entfernt werden. Sämtliche dieser beschriebenen Vorgänge können vorteilhafterweise während eines einzigen Bearbeitungszuges über die Haut ausgeführt werden.

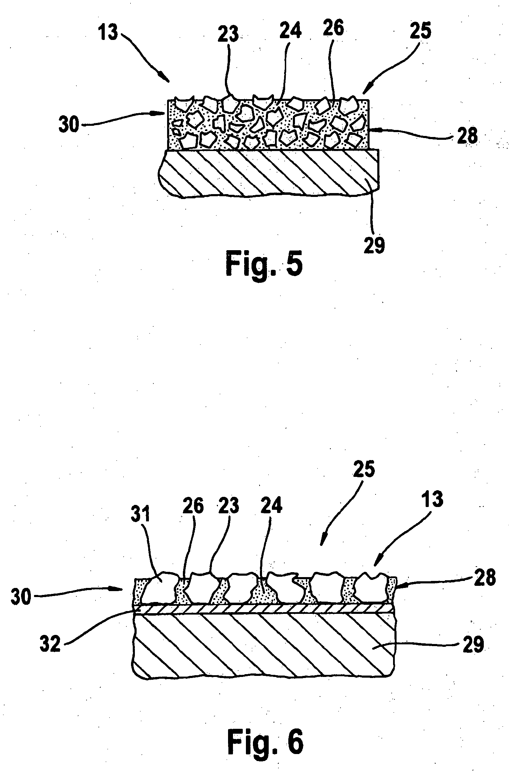

Vorteilhafterweise umfasst der Bearbeitungskorpus 13 des Hautbearbeitungswerkzeuges 21 eine als Schleiffläche ausgebildete Bearbeitungsfläche 22, die mit einer Applikationseinrichtung 25 zur Abgabe eines Applikationsstoffes versehen ist. Die Bearbeitungsfläche 22 besitzt dabei eine poröse Oberfläche mit einer geeigneten Rauhtiefe vorzugsweise im Bereich von 30 Rz bis 70 Rz, die eine abrasive Wirkung entfaltet und für ein schonendes und trotzdem effizientes Peeling geeignet ist. Wie Figur 5 zeigt, umfasst die Bearbeitungsfläche 22 dabei eine Vielzahl von geometrisch unregelmäßig geformten Vorsprüngen 23 in einer dichten, jedoch zufällig verstreuten Anordnung über die Fläche, wobei zwischen den Vorsprüngen 23 Poren 24 gebildet sind. Nach einer bevorzugten Ausführung der Erfindung wird die Oberfläche der Bearbeitungsfläche 22 mit einem Metallpulver und/oder mit einem Keramikpulver plasmabeschichtet oder flammbeschichtet, wodurch sich die in Figur 5 gezeigte Oberflächenstruktur ergibt. Das bei dem Flammspritz- oder Plasmabeschichtungsprozess verwendete Metallpulver oder Keramikpulver kann vorteilhafterweise eine Korngröße zwischen 10 µm und 100 µm besitzen. Die hierdurch hergestellte Schleifbeschichtung 30 ist vorteilhafterweise auf einem Träger 29 aufgebracht, der aus Kunststoff gefertigt sein kann.

Alternativ zu einer solchen plasmabeschichteten bzw. flammbeschichteten Oberfläche kann auch eine schleifpapierähnliche Schleifbeschichtung 30 auf den Träger 29 aufgebracht sein, wie dies Figur 6 zeigt. Die Schleifbesichtung 30 umfasst hierbei eine Vielzahl von geometrisch unregelmäßig geformten Schleifkörnern 31 aus einem geeigneten Schleifkornmaterial, die in der gezeichneten Ausführungsform auf einer Zwischenschicht aus Papier 32 aufgeklebt sein können, welches wiederum auf dem Träger 29 befestigt, beispielsweise aufgeklebt ist. Nach einer alternativen, nicht eigens gezeichneten Ausführung der Erfindung kann die Schleifbeschichtung 30 mit den Schleifkörnern 31 jedoch auch direkt auf den Träger 29 aufgeklebt sein, wodurch eine separate Zwischenschicht aus Papier eingespart wird. Um die gewünschte Rauhtiefe bzw. die gewünschte abrasive Wirkung zu erreichen, wird für die Schleifkörner vorzugsweise eine Körnung zwischen 100 und 1200 eingesetzt.

Wie auch Figur 6 zeigt, ist auch bei der schleifpapierähnlichen Ausbildung der Bearbeitungsfläche 22 zwischen den Schleifkörnern 31 eine Vielzahl von Poren 24 vorgesehen. In den Poren 24 der porösen Bearbeitungsfläche 22 ist dabei ein Applikationsstoff eingelagert, der die genannten Poren 24 im wesentlichen verschließt und hierdurch das Einnisten von Verunreinigungen, Bakterien und Schmutz verhindert. Aus den Poren 24 heraus kann der Applikationsstoff dabei nach und nach bei der Bearbeitung der Haut an diese abgegeben werden. Die Applikationsstoffeinlagerungen 26 in den Poren 24 bilden dabei sozusagen einen Applikationsstoffspeicher 28 für eine kontinuierliche, langsame Abgabe des Applikationsstoffes.

Der Applikationsstoff kann dabei in einer Emulsion oder in flüssiger Form in und auf die poröse Oberfläche des Bearbeitungskorpus 13 gebracht werden, wobei sie von dem hydrophil ausgebildeten Bearbeitungskorpus 13 aufgenommen werden. Der Auftrag kann dabei durch Aufsprühen oder Tauchen erfolgen, insbesondere bei der Herstellung des Bearbeitungskorpus 13 mit einer schleifpapierähnlichen Schleifbeschichtung 30 kann die Applikationsstoffbeschichtung auch mittels einer Auftragswalze oder durch einen Druckprozess erfolgen.

## Patentansprüche

1. Hautbearbeitungswerkzeug zum Hautpeeling mit
• zumindest einem Bearbeitungskorpus (13), der eine Bearbeitungsfläche (22) mit einer Vielzahl von Bearbeitungsvorsprüngen (23) aufweist,
• eine Applikationseinrichtung (25) zum Aufbringen eines Applikationsstoffs auf die von dem Bearbeitungskorpus (13) bearbeitbare Haut
wobei die Applikationseinrichtung (25) einen Applikationsstoffspeicher (28) in und/oder auf der Bearbeitungsfläche (22) des Bearbeitungskorpus (13) aufweist und der Applikationsstoffspeicher (28) zumindest teilweise durch
Applikationsstoffeinlagerungen (26), die Oberflächensenken, insbesondere Poren (24), zwischen den Bearbeitungsvorsprüngen (23) verschließen, gebildet ist, **dadurch gekennzeichnet, dass** das Hautbearbeitungswerkzeug als Geräteaufsatz (11) für ein Haarentfernungs- und/oder - schnittgerät (1) ausgebildet ist und wobei der Geräteaufsatz (11) an einen Bearbeitungskopf (20) des Geräts (1) angepasste, formschlüssige Befestigungsmittel (27) aufweist.

2. Hautbearbeitungswerkzeug nach dem vorhergehenden Anspruch, wobei die Applikationseinrichtung (25) derart ausgebildet ist, dass die Abgabe des Applikationsstoffs im Bereich der Bearbeitungsfläche (22) erfolgt.

3. Hautbearbeitungswerkzeug nach einem der vorhergehenden Ansprüche, wobei der Applikationsstoff ein Hautpflegemittel vorzugsweise enthaltend einen Feuchtigkeitsspender, ein Öl, Aloe Vera und/oder ein Coenzym umfasst.

4. Hautbearbeitungswerkzeug nach einem der vorhergehenden Ansprüche, wobei der Applikationsstoff einen antibakteriellen Wirkstoff enthält.

5. Hautbearbeitungswerkzeug nach einem der vorhergehenden Ansprüche, wobei der Applikationsstoff abreibbar auf die Bearbeitungsfläche (22) aufgebracht ist.

6. Hautbearbeitungswerkzeug nach einem der vorhergehenden Ansprüche, wobei die Bearbeitungsfläche (22) als Schleifoberfläche ausgebildet ist, die eine Vielzahl von unregelmäßig geformten Schleifvorsprüngen (23) besitzt.

7. Hautbearbeitungswerkzeug nach einem der vorhergehenden Ansprüche, wobei der Bearbeitungskorpus (13) einen Träger (29) aufweist, auf dem eine Schleifbeschichtung (30) aufgebracht ist.

8. Hautbearbeitungswerkzeug nach dem vorhergehenden Anspruch, wobei die Schleifbeschichtung (30) als Plasmabeschichtung mit einem Keramik- und/oder Metallpulver ausgebildet ist.

9. Hautbearbeitungswerkzeug nach Anspruch 7, wobei die Schleifbeschichtung (30) als Flammbeschichtung mit einem Keramik- und/oder Metallpulver ausgebildet ist.

10. Hautbearbeitungswerkzeug nach Anspruch 7, wobei die Schleifbeschichtung (30) eine Vielzahl von Schleifkörnern (31) aufweist, die an dem Träger (29) befestigt, insbesondere festgeklebt sind.

11. Hautbearbeitungswerkzeug nach dem vorhergehenden Anspruch, wobei die Schleifbeschichtung (30) eine Körnung im Bereich von 100 bis 1200 aufweist.

12. Hautbearbeitungswerkzeug nach einem der vorhergehenden Ansprüche, wobei die Schleifbeschichtung (30) eine Rauhtiefe von 5 Rz bis 100 Rz, vorzugsweise 30 Rz bis 70 Rz, besitzt.

13. Hautbearbeitungswerkzeug nach Anspruch 1, wobei der Bearbeitungskorpus (13) beweglich gelagert ist und Kupplungsmittel zum Ankuppeln an einen Geräteantrieb aufweist.

14. Elektrohandgerät des persönlichen Bedarfs, ausgebildet als Haarentfernungs- und/oder - schnittgerät, mit einem Hautbearbeitungswerkzeug (21) nach einem der vorhergehenden Ansprüche.

15. Elektrohandgerät nach dem vorhergehenden Anspruch, wobei der Bearbeitungskorpus (13) mit einem Antrieb koppelbar und von diesem oszillierend, rotierend oder vibrierend, vorzugsweise translatorisch oszillierend antreibbar ist.

16. Verfahren zur Herstellung eines Hautbearbeitungswerkzeugs (21) mit zumindest einer Bearbeitungsfläche (22) nach einem der Ansprüche 1 bis 16, **dadurch gekennzeichnet, dass** ein Applikationsstoff auf der und/oder in der Bearbeitungsfläche (22) eingelagert wird.

17. Verfahren nach dem vorhergehenden Anspruch, wobei der Applikationsstoff durch Sprühtrocknen einer Emulsion oder Dispersion aufgetragen wird.

18. Verfahren nach Anspruch 16, wobei der Applikationsstoff durch Tauchen aufgebracht wird.

19. Verfahren nach Anspruch 16, wobei der Applikationsstoff aufgewalzt wird.

20. Verfahren nach Anspruch 16, wobei der Applikationsstoff durch ein Druckverfahren aufgebracht wird.

21. Verfahren nach einem der Ansprüche 16 bis 20, wobei vor dem Aufbringen des Applikationsstoffs die Bearbeitungsfläche (22) mit einem Metallpulver und/oder einem Keramikpulver plasmabeschichtet wird.

22. Verfahren nach einem der Ansprüche 16 bis 20, wobei vor dem Aufbringen des Applikationsstoffs die Bearbeitungsfläche (22) mit einem Metallpulver und/oder einem Keramikpulver flammbeschichtet wird.

23. Verfahren nach einem der Ansprüche 16 bis 20, wobei die Bearbeitungsfläche (22) vor dem Aufbringen des Applikationsstoffs mit einer Schleifkornschicht beschichtet wird.

24. Verfahren nach einem der Ansprüche 21 bis 23, wobei der Applikationsstoff in Poren (24) zwischen Bearbeitungsvorsprüngen (23) der Bearbeitungsfläche (22) eingelagert wird.

## Claims

1. A skin treatment tool for skin peeling comprising
• at least a treatment body (13) that has a treatment surface (22) with a plurality of treatment protrusions (23),
• an application system (25) for applying an application material onto the skin that can be treated by the treatment body (13)
the application system (25) having an application material reservoir (28) into and/or onto the treatment surface (22) of the treatment body (13) and the application material reservoir (28) being at least partially formed by application material deposits (26) that seal surface recesses, in particular pores (24), between the treatment protrusions (23), **characterized in that** the skin treatment tool is designed as a device attachment (11) for a hair removal and/or cutting device (1) and wherein the device attachment (11) has positive-locking fastening mechanisms (27) adapted to a treatment head (20) of the device (1).

2. The skin treatment tool according to the preceding claim, wherein the application system (25) is designed in such a way that the application material is dispensed in the region of the treatment surface (22).

3. The skin treatment tool according to the preceding claim, wherein the application material comprises a skincare product preferably containing a moisturizer, an oil, aloe vera and/or a coenzyme.

4. The skin treatment tool according to one of the preceding claims, wherein the application material contains an antibacterial active ingredient.

5. The skin treatment tool according to one of the preceding claims, wherein the application material is applied to the treatment surface (22) such that it can be rubbed off.

6. The skin treatment tool according to one of the preceding claims, wherein the treatment surface (22) is designed as an abrasive surface that has a plurality of irregularly shaped abrasive projections (23).

7. The skin treatment tool according to one of the preceding claims, wherein the treatment body (13) has a carrier (29) on which an abrasive coating (30) is applied.

8. The skin treatment tool according to one of the preceding claims, wherein the abrasive coating (30) is designed as a plasma coating with a ceramic and/or metal powder.

9. The skin treatment tool according to claim 7, wherein the abrasive coating (30) is designed as a flame coating with a ceramic and/or metal powder.

10. The skin treatment tool according to claim 7, wherein the abrasive coating (30) has a plurality of abrasive grains (31), which are attached, in particular glued, to the carrier (29).

11. The skin treatment tool according to the preceding claim, wherein the abrasive coating (30) has a grain size in the range from 100 to 1200.

12. The skin treatment tool according to one of the preceding claims, wherein the abrasive coating (30) has a surface roughness of 5 Rz to 100 Rz, preferably 30 Rz to 70 Rz.

13. The skin treatment tool according to claim 1, wherein the treatment body (13) is mounted in a movable manner and has a coupling means for coupling it to a drive of the device.

14. An electrically operated hand-held device for personal use, designed as a hair removal and/or cutting device, having a skin treatment tool (21) according to one of the preceding claims.

15. The electrically operated hand-held device according to the preceding claim, wherein the treatment body (13) can be joined to a drive and driven by the same in an oscillating, rotating or vibrating, preferably translational oscillating, manner.

16. A method for manufacturing a skin treatment tool (21) having at least a treatment surface (22) according to one of the claims 1 to 16, **characterized in that** an application material is stored on and/or in the treatment surface (22).

17. The method according to one of the preceding claims, wherein the application material is applied by spray drying an emulsion or dispersion.

18. The method according to claim 16, wherein the application material is applied by means of immersion.

19. The method according to claim 16, wherein the application material is rolled on.

20. The method according to claim 16, wherein the application material is applied using a printing method.

21. The method according to one of the claims 16 to 20, wherein the treatment surface (22) is plasma-coated with a metal powder and/or a ceramic powder before the application material is applied.

22. The method according to one of the claims 16 to 20, wherein the treatment surface (22) is flame-coated with a metal powder and/or a ceramic powder before the application material is applied.

23. The method according to one of the claims 16 to 20, wherein the treatment surface (22) is coated with a layer of abrasive grains before the application material is applied.

24. The method according to one of the claims 21 to 23, wherein the application material is stored in pores (24) between treatment protrusions (23) of the treatment surface (22).

## Revendications

1. Outil de traitement de la peau destiné au gommage de la peau, comprenant
• au moins un corps (13) de traitement, qui présente une surface (22) de traitement dotée d'une pluralité de saillies (23) de traitement,
• un dispositif (25) d'application destiné à appliquer une substance d'application sur la peau traitée par le corps (13) de traitement
dans lequel le dispositif (25) d'application présente un réservoir de substance d'application (28) dans et/ou sur la surface (22) de traitement du corps (13) de traitement et le réservoir de substance d'application (28) est formé au moins partiellement par des entreposages (26) de substance d'application, qui ferment des creux de surface, notamment des pores (24), entre les saillies (23) de traitement, **caractérisé en ce que** l'outil de traitement de la peau est formé comme un embout (11) d'appareil pour un appareil (1) de coupe des poils ou d'épilation et dans lequel l'embout (11) d'appareil présente un moyen (27) de fixation à correspondance de forme adapté à une tête (20) de traitement de l'appareil (1).

2. Outil de traitement de la peau selon la revendication précédente, dans lequel le dispositif (25) d'application est formé de telle sorte que la délivrance de la substance d'application s'effectue dans la région de la surface (22) de traitement.

3. Outil de traitement de la peau selon la revendication précédente, dans lequel la substance d'application comprend un agent de soin de la peau, de préférence contenant un hydratant, une huile, de l'aloe vera et/ou une co-enzyme.

4. Outil de traitement de la peau selon l'une quelconque des revendications précédentes, dans lequel la substance d'application contient un agent actif antibactérien.

5. Outil de traitement de la peau selon l'une quelconque des revendications précédentes, dans lequel la substance d'application est appliquée sur la surface (22) de traitement de façon à pouvoir être abrasée.

6. Outil de traitement de la peau selon l'une quelconque des revendications précédentes, dans lequel la surface (22) de traitement est formée comme une surface abrasive, qui possède une pluralité de saillies (23) abrasives formées de manière irrégulière.

7. Outil de traitement de la peau selon l'une quelconque des revendications précédentes, dans lequel le corps (13) de traitement présente un support (29) sur lequel un revêtement abrasif (30) est appliqué.

8. Outil de traitement de la peau selon la revendication précédente, dans lequel le revêtement abrasif (30) est formé comme un revêtement au plasma avec une poudre métallique et/ou céramique.

9. Outil de traitement de la peau selon la revendication 7, dans lequel le revêtement abrasif (30) est formé comme un revêtement à la flamme avec une poudre métallique et/ou céramique.

10. Outil de traitement de la peau selon la revendication 7, dans lequel le revêtement abrasif (30) présente une pluralité de grains abrasifs (31), qui sont fixés sur le support (29), en particulier collés.

11. Outil de traitement de la peau selon la revendication précédente, dans lequel le revêtement abrasif (30) présente une granulation dans la plage de 100 à 1200.

12. Outil de traitement de la peau selon l'une quelconque des revendications précédentes, dans lequel le revêtement abrasif (30) possède une profondeur de rugosité allant de 5 Rz à 100 Rz, de préférence de 30 Rz à 70 Rz.

13. Outil de traitement de la peau selon la revendication 1, dans lequel le corps (13) de traitement est placé de façon mobile et présente un moyen d'accouplement destiné à l'accouplement sur un entraînement d'appareil.

14. Appareil électrique manuel à usage personnel, formé comme un appareil de coupe des poils et/ou d'épilation, avec un outil (21) de traitement de la peau selon l'une quelconque des revendications précédentes.

15. Appareil électrique manuel selon la revendication précédente, dans lequel le corps (13) de traitement peut être accouplé avec un entraînement et peut être entraîné par celui-ci de façon oscillante, rotative ou vibratoire, de préférence de façon oscillante en translation.

16. Procédé de fabrication d'un outil (21) de traitement de la peau avec au moins une surface (22) de traitement selon une des revendications 1 à 16, **caractérisé en ce qu'**une substance d'application est stockée sur et/ou dans la surface (22) de traitement.

17. Procédé selon la revendication précédente, dans lequel la substance d'application est appliquée par séchage par pulvérisation d'une émulsion ou d'une dispersion.

18. Procédé selon la revendication 16, dans lequel la substance d'application est appliquée par immersion.

19. Procédé selon la revendication 16, dans lequel la substance d'application est appliquée par laminage.

20. Procédé selon la revendication 16, dans lequel la substance d'application est appliquée par un procédé de pression.

21. Procédé selon une des revendications 16 à 20, dans lequel, avant l'application de la substance d'application, la surface (22) de traitement est revêtue au plasma d'une poudre métallique et/ou d'une poudre céramique.

22. Procédé selon une des revendications 16 à 20, dans lequel, avant l'application de la substance d'application, la surface (22) de traitement est revêtue à la flamme d'une poudre métallique et/ou d'une poudre céramique.

23. Procédé selon une des revendications 16 à 20, dans lequel la surface (22) de traitement, avant l'application de la substance d'application, est revêtue avec une couche de grains abrasifs.

24. Procédé selon une des revendications 21 à 23, dans lequel la substance d'application est stockée dans des pores (24) entre les saillies (23) de traitement de la surface (22) de traitement.
